Europäisches Patentamt

European Patent Office

Office européen des brevets

(11) Publication number: **0 121 157**
**B1**

# EUROPEAN PATENT SPECIFICATION

(45) Date of publication of patent specification: **14.06.89**

(21) Application number: **84102712.1**

(22) Date of filing: **13.03.84**

(51) Int. Cl.⁴: **C 12 N 15/00,** C 12 P 21/02, C 07 K 1/00, C 12 N 1/20 // (C12N1/20, C12R1:19)

(54) Novel human interferon-gamma polypeptide.

(30) Priority: **14.03.83 JP 42042/83**

(43) Date of publication of application:
**10.10.84 Bulletin 84/41**

(45) Publication of the grant of the patent:
**14.06.89 Bulletin 89/24**

(84) Designated Contracting States:
**AT BE CH DE FR GB IT LI NL SE**

(56) References cited:
EP-A-0 063 482
EP-A-0 077 670
EP-A-0 083 069
EP-A-0 083 069
EP-A-0 083 777
EP-A-0 088 540
EP-A-0 089 676
EP-A-0 095 350
EP-A-0 099 084

NATURE, vol. 295, 11 Feb 1982, Macmillan Journals Ltd; P.W.GRAY et al.: "Expression of human immune interferon cDNA in E. coli and monkey cells", pp. 503-508

(73) Proprietor: **KYOWA HAKKO KOGYO CO., LTD.**
**Ohtemachi Bldg., 6-1 Ohtemachi I-chome**
**Chiyoda-ku Tokyo 100 (JP)**
(73) Proprietor: **Juridical Foundation Japanese**
**Foundation for Cancer Research**
**37-1, Kami-Ikebukuro 1-chome Toshima-ku**
**Tokyo (JP)**

(72) Inventor: **Itoh, Seiga**
**218-14, Aihara**
**Sagamihara-shi Kanagawa-ken (JP)**
Inventor: **Nishi, Tatsunari**
**5-19-24, Okura**
**Setagaya-ku Tokyo (JP)**
Inventor: **Taniguchi, Tadatsugu**
**Yuparesu Tagara 303 4-27-12, Tagara**
**Nerima-ku Tokyo (JP)**
Inventor: **Sugano, Haruo**
**4-8-13, Minami-Ogikubo**
**Suginami-ku Tokyo (JP)**

(74) Representative: **Vossius & Partner**
**Siebertstrasse 4 P.O. Box 86 07 67**
**D-8000 München 86 (DE)**

(56) References cited:

NUCLEIC ACIDS RESEARCH, vol. 10, no. 12,
1982, IRL Press Ltd., Oxford (GB); R.DERYNCK
et al.: "Human interferon gamma is encoded by
a single class of mRNA", pp. 3605-3615

NATURE, vol. 294, 10 Dec 1981, Macmillan
Journals Ltd.; H.M.SHEPARD et al.: "A single
amino acid change in IFN-beta 1 abolishes it's
antiviral activity", pp. 563-565

NUCLEIC ACIDS RESEARCH, Symposium Series,
no. 11, 1982, IRL Press Ltd., Oxford, GB;
S.TANAKA et al.: "Expression in Escherichia
coli of chemically synthesized gene for a
human immune interferon", pp. 29-32

CHEMICAL ABSTRACTS, vol. 97, 1982,
Columbus, OH (US); R.DEVOS et al.:
"Molecular cloning of human interferon cDNA
and it's expression in eukaryotic cells", p. 219,
no. 18357j

CHEMICAL ABSTRACTS, vol. 98, 1983,
Columbus, OH (US); C.C.SIMONSEN et al.:
"Plasmid-directed synthesis of human immune
interferon in E. coli and monkey cells", p. 115,
no. 1188c

**Description**

Interferons (referred to as IFN hereinafter) so far known can be classified into three large groups, i.e. IFN—α, IFN—β and IFN—γ. IFN—α is mainly produced by leukocytes, IFN—β by fibroblasts and IFN—γ by T-lymphocytes. These IFNs have been noted as biologically active substances having antiviral activity, activating activities upon natural killer cells and macrophages, antitumor activity, and the like. However, the conventional methods for obtaining IFNs by isolation from leukocytes and cultured cells cannot provide them sufficiently.

Recombinant DNA technology has now been developed to the extent that the mass production of substances such as IFN which are produced only in minute quantities in cells of higher animals has become possible by using transformed microorganisms. For example, mRNAs of IFN—β and IFN—α were respectively isolated from cells and cDNA complementary to the mRNA were synthesized enzymatically, inserted into a suitable plasmid as a double stranded DNA and cloned in *Escherichia coli* [Taniguchi et al., Proc. Jap. Acad., Vol. 55(B), 464—469 (1979), Nagata et al., Nature, Vol. 284, 316—320 (1980)].

As for IFN—γ, there has been a report that it has a stronger cell-inhibiting activity than other IFNs based on experiments using animal cells [B.Y. Rubin and S.L. Gupta, Proc., Natl. Acad. Sci., USA, Vol. 77, 5928—5932 (1980)]. Furthermore, cloning of an IFN—γ DNA in *Escherichia coli* and determination of its base sequence was recently reported [P.W. Gray et al., Nature, Vol. 295, 503—508 (1982) and R. Devos et al., Nucleic Acids Research, Vol., 10, No. 8, 2487—2501 (1982)].

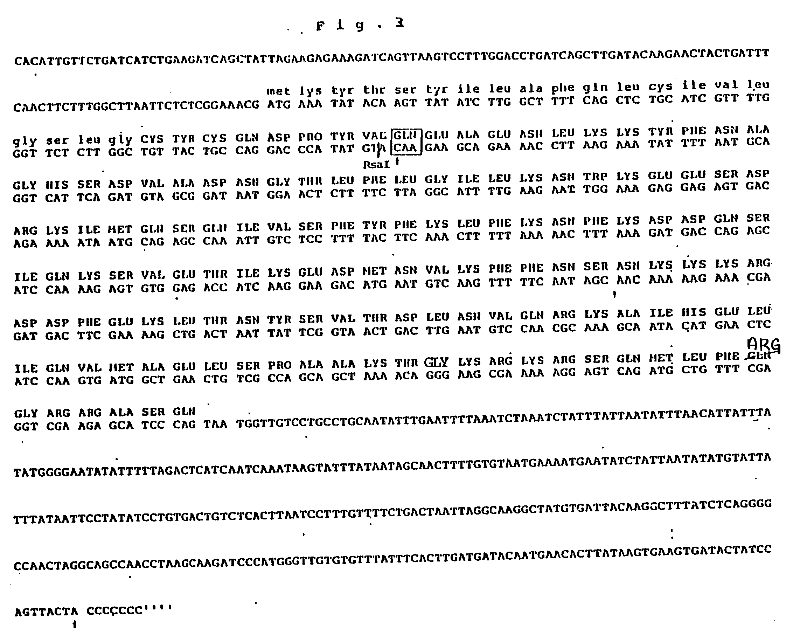

It is an object of the present invention to provide novel recombinant plasmids containing a tryptophan promotor and downstream thereof a DNA fragment encoding a human IFN—γ polypetide having the aminoacid sequence illustrated in Figure 3 and microorganisms transformed therewith for the production of a human IFN—γ polypeptide having the aminoacid sequence given in Figure 3.

Brief Description of the Drawings
Fig. 1 is a flow chart illustrating the construction of plasmid pGC—7.
Fig. 2 is a flow chart illustrating the construction of plasmid pGKA—2.
Fig. 3 illustrates the base sequence around the human IFN—γ DNA in plasmid pGKA—2.
Fig. 4 (A) illustrates the structure of plasmid pKYP—1 and (B) illustrates that of pKYP—5.
Fig. 5 is a flow chart illustrating the construction of pKYP—5.
Fig. 6 is a flow chart illustrating the construction of pKYP—10.
Fig. 7 (A) is a flow chart illustrating the construction of pKYP—11 and (B) illustrates the structure of pKYP—12.

The construction of the recombinant plasmids is carried out in the following manner.

As the novel human IFN—γ DNA, pIFNγ—G4 which was cloned by the present inventors is used. *Escherichia coli* containing pIFNγ—G4 has been deposited with the American Type Culture Collection, U.S.A. under accession number ATCC 39123.

The base sequence of the IFN—γ DNA in pIFN—G4 was determined by the Maxam-Gilbert chemical procedure [Proc. Natl., Acad. Sci., Vol. 74, 499—560 (1977)] and is illustrated in Fig. 3.

A comparison of the human IFN—γcDNA in pIFNγ—G4 and the known IFN—γcDNA [P.W. Gray et al., Nature, Vol. 295, 503—508 (1982)] reveals the following. The first base of the triplet coding for the ninth amino acid from the N-terminal of the mature human IFN—γ polypeptide, lysine, in the known cDNA is adenine [Gray et al., Nature, Vol. 295, 503—508 (1982)]. On the other hand, as the corresponding base in the pIFNγ—G4cDNA is cytosine, the ninth amino acid from the N-terminal of the human IFN—γ polypeptide encoded by the pIFNγ—G4cDNA is glutamine, and not lysine. Therefore, it is apparent that pIFNγ—G4 codes for a novel human IFN—γ polypeptide.

Further, pIFNγ—G4 has a RsaI restriction site as the 25th base is C. That is, the sequence of the 22nd to 25th bases from 5'-terminus of the IFNγ—DNA in pIFNγ—G4 is GTAC and RsaI cuts the DNA at the position illustrated with the arrow (GT ↓ AC).

Since none of the known IFN—γ genes have a RsaI site in a DNA region coding for IFN—γ polypeptide, it is apparent that pIFNγ—G4 contains a DNA coding for a novel human IFN—γ polypeptide.

Therefore, recombinant plasmids constructed using IFN—γ DNA of pIFN—γ.G4 are all new recombinant plasmids.

As the plasmid to incorporate IFN—γDNA, any plasmid can be used so long as the DNA incorporated therein can be expressed in *Escherichia col*. Preferably, a plasmid wherein a foreign DNA can be inserted downstream from a suitable promoter such as trp promoter or lac promoter and the length of the sequence between Shine-Dalgarno sequence (referred to as SD sequence hereinafter) and the initiation site of translation is adjusted to, for example, 6—18 base pairs is employed. Preferred examples are pKYP—10, pKYP—11 and pKYP—12 which were constructed by the present inventors [Japanese Patent Application 213193/81 (Japanese Published Unexamined Patent Application 110600/83)]. These expression vectors prepared by the method illustrated in Reference Example 1 contain tryptophan promoters (referred to as Ptrp hereinafter).

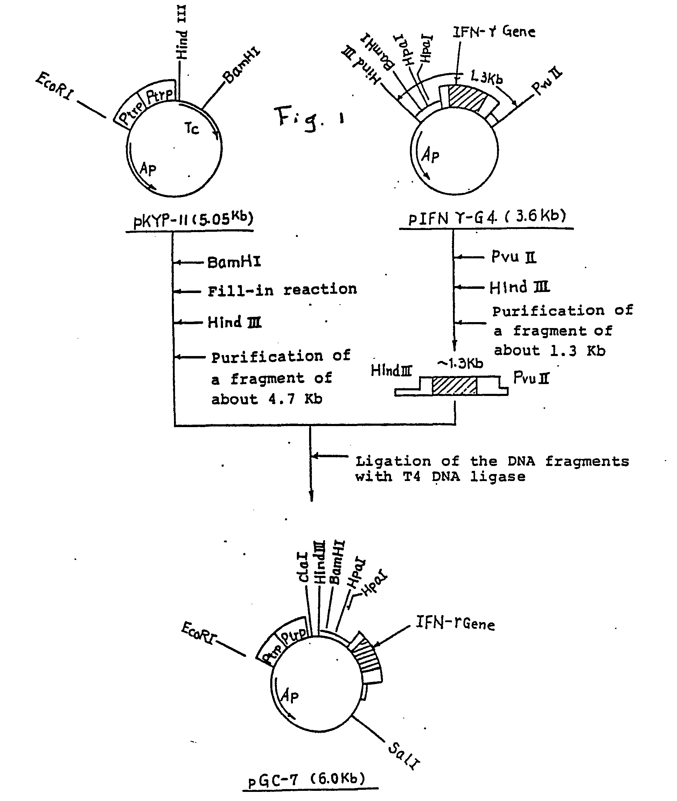

Recombinant plasmid pGC—7 is prepared from pKYP—11 and pIFNγ—G4 as illustrated in Fig. 1. That is, pIFNγ—G4 is digested with PvuII and HindIII and the digest is purified by low-gelling-temperature agarose gel electrophoresis [L. Wieslander, Analytical Biochemistry *98*, 305 (1979)]. The resulting fragment

of pIFNγ—G4 is inserted into pKYP—11 at the HindIII — BamHI site to obtain pGC—7.

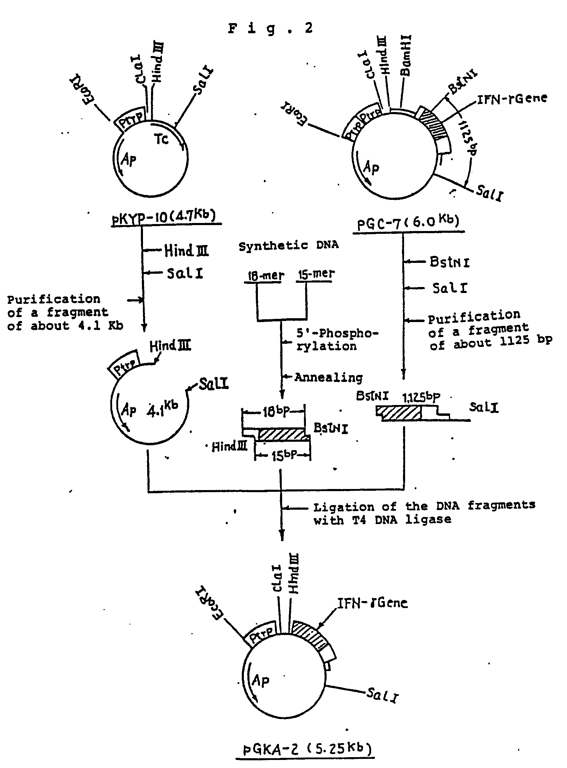

Thereafter, as illustrated in Fig. 2 pGC—7 is cut with BstNI and SalI and IFN—γ DNA is purified by low-gelling-temperature agarose gel electrophoresis. The IFN—γ DNA is cloned together with a synthetic DnA into the HindIII — SalI site of the expression vector pKYP—10 harboring Ptrp to obtain recombinant plasmid pGKA—2.

The IFN—γ polypeptide of the present invention is prepared in the following manner.

*Escherichia coli* K—12 HB101 [S. N. Cohen et al., Proc., Natl. Acad. Sci., Vol. 69, 2110 (1972)] is transformed with pGKA—2 and an *Escherichia coli* strain containing pGKA—2 is selected from colonies resistant to ampicillin (referred to as Ap^R hereinafter). The *Escherichia coli* strain containing pGKA—2 is cultured in a medium to produce IFN—γ polypeptide in the cells.

As the medium, either a synthetic medium or a natural medium can be used as long as it is suitable for the growth *Escherichia coli* and the production of IFN—γ polypeptide.

As a carbon source, glucose, fructose, lactose, glycerol, mannitol, sorbitol, etc. may be used.

As a nitrogen source, $NH_4Cl$, $(NH_4)_2SO_4$, casamino acid, yeast extract, polypeptone, meat extract, bactotryptone, corn steep liquor, etc. may be used.

In addition, nutrients such as $NH_2HPO_4$, $K_2HPO_4$, $KH_2PO_4$, NaCl, $MgSO_4$, vitamine $B_1$ and $MgCl_2$ may be used.

Culturing is carried out at pH 5.5—8.5 and at 18—40°C with aeration and stirring.

After culturing for 5—90 hours, the human IFN—γ polypeptide is accumulated in the cultured cells. The collected cells are treated with lysozyme, disrupted by repeated freezing and melting and subjected to centrifugation. The thus obtained supernatant fluid is subjected to extraction according to conventional methods for extraction of polypeptides to recover the polypeptide.

Determination of human IFN—γ is carried out according to the method of Armstrong [J.A. Armstrong et al., Appl. Microbiol., Vol. 21, 723—725 (1971)].

Certain specific embodiments of the present invention are illustrated by the following examples.

## Example 1

Insertion of human IFN—γ DNA into the expression vector pKYP—11:

In this example, 6 µg of plasmid pIFNγ—G4 [3.6 kilobases (referred to as Kb hereinafter)] was dissolved in 50 µl of a solution containing 20 mM Tris—HCl (pH 7.5), 10 mM $MgCl_2$, 10 mM dithiothreitol and 50 mM NaCl. Then, 12 units each of restriction enzymes PvuII (product of Takara Shuzo Co.; the restriction enzymes hereinafter are all products of Takara Shuzo Co., unless otherwise specified) and HindIII were added and digestion reaction was carried out at 37°C for 4 hours. The reaction solution was heated at 65°C for 7 minutes to inactivate the enzymes and subjected to purification by low-gelling-temperature agarose gel electrophoresis to obtain 1.2 µg of a DNA fragment containing human IFN—γ DNA of 1.3 Kb.

Separately, 4 µg of pKYP—11 was dissolved in 40 µl of a solution containing 20 mM Tris—HCl (pH 7.5), 10 mM $MgCl_2$, 10 mM dithiothreitol and 50 mM NaCl. 8 units of BamHI was added and digestion reaction was carried out at 37°C for 3 hours. The reaction solution was heated at 65°C for 5 minutes to inactivate the enzyme. Thereafter, 30 µm each of dATP, dCTP, dGTP and dTTP were added and 8 units of *Escherichia coli* DNA polymerase I (Klenow fragment, product of New England Biolabs, 1 µl) was added. Fill-in reaction was carried out at 15°C for 1 hour and the reaction solution was heated at 68°C for 15 minutes to inactivate the DNA polymerase I. 10 units of HindIII was added and digestion reaction was carried out at 37°C for 3 hours, followed by heating at 65°C for 5 minutes to inactivate the HindIII. The digestion reaction solution of the plasmid pKYP—11 was subjected to purification by low-gelling-temperature agarose gel electrophoresis to obtain about 2.5 µg of a DNA fragment of about 7.7 Kb containing Ptrp.

Then, 0.5 µg of the DNA fragment of 1.3 Kb containing human IFN—γ DNA and 1.0 µg of the DNA fragment of about 4.7 Kb containing Ptrp, which was obtained from the plasmid pKYP—11, were dissolved in 20 µl of a solution containing 20 mM Tris—HCl (pH 7.5), 6 mM $MgCl_2$, 5 mM dithiothreitol and 500 µM ATP, and 4 units of T4 DNA ligase (product of New England Biolabs) was added. Ligation reaction was carried out at 4°C for 18 hours, and *Escherichia coli* HB101 was transformed with the obtained redcombinant plasmid mixture by conventional technique to obtain an Ap^R colony. A plasmid, pGC—7 illustrated in Fig. 1 was separated from the culture broth of the colony. The structure of pGC—7 was confirmed by digestion with HindIII, BamHI, HpaI, SalI, EcoRI and ClaI and agarose gel electrophoresis. *Escherichia coli* strain containing pGC—7 has been deposited with the Fermentation Research Institute, Agency of Industrial Science and Technology (referred to as FERM hereinafter) as *Escherichia coli* IGC—7 (FERM P—6814) (FERM BP—497).

## Example 2

Construction of recombinant plasmid pGKA—2:

In this example, 6 µg of the pGC—7 DNA obtained in Example 1 was dissolved in 50 µl of a solution containing 20 mM Tris—HCl (pH 7.5), 10 mM $MgCl_2$, 10 mM dithiothreitol and 10 mM NaCl, and 12 units of BstNI (product of New England Biolabs) was added. Reaction was carried out at 60°C for 3 hours. Then, 150 mM NaCl and 8 units of SalI were added and digestion reaction was carried out at 37°C for 3 hours. The reaction solution was again heated at 65°C for 5 minutes to inactivate the SalI and subjected to purification by low-gelling-temperature agarose gel electrophoresis to obtain about 0.8 µg of a DNA fragment of about

EP 0 121 157 B1

1,125 base pairs (referred to as bp hereinafter) containing a large portion of the human IFN—γ DNA.

Separately, 3 μg of pKYP—10 was dissolved in 40 μl of a solution containing 20 mM Tris—HCl (pH 7.5), 10 mM MgCl₂, 10 mM dithiothreitol and 100 mM NaCl. 6 units each of HindIII and SalI were added and digestion reaction was carried out at 37°C for 3 hours. The reaction solution was heated at 65°C for 5 minutes to inactivate HindIII and SalI and subjected to purification by low-gelling-temperature agarose gel electrophoresis to obtain about 1.8 μg of a DNA fragment of about 4.1 Kb containing Ptrp.

The N-terminal amino acid of the mature human IFN—γ polypeptide is cystein (Cys). In order to express mature IFN—γ DNA, it is necessary to furnish an initiation codon (ATG) just before the 5′-terminal codon TGT (Cys) and further to adjust the length of the sequence between SD-sequence and ATG to a suitable length of 6—18 bp. Therefore, the following DNA linker was synthesized.

```
HindIII     M e t C y s T y r C y s   BstNI
        5' AGCT T|A T G|T G T T A C T G C C|3'     (18-mer)
              3'|A T A C A C A A T G A C G G T|5'  (15-mer)
```

Two single chain DNAs of 18-mer and 15-mer were synthesized by a conventional tri-ester method [R. Crea, et al.: Proc. Natl. Acad. Sci., 75, 5765 (1978)]. Then, 2 μg each of the 18-mer and 15-mer DNAs were dissolved in 20 μl of a solution containing 50 mM Tris-HCl (pH 7.5), 10 mM MgCl₂, 5 mM dithiothreitol, 0.1 mM EDTA and 1 mM ATP. 30 units of T4 polynucleotide kinase (product of Boehringer Mannheim) was added and phosphorylation reaction was carried out at 37°C for 60 minutes.

Then, 2 μg each of phosphorylated 18-mer and 15-mer DNAs were mixed and the mixture was heated at 70°C for 5 minutes and allowed to stand at room temperature for annealing to obtain the DNA linker having the structure given above.

0.4 μg of the BstNI—SalI fragment of 1,125 bp obtained above and derived from pGC—7 and 1.0 μg of the DNA fragment of 4.1 Kb obtained by digestion of the expression vector pKYP—10 with HindIII and SalI were dissolved in 25 μl of a solution containing 20 mM Tris—HCl (pH 7.5), 6 mM MgCl₂, 5 mM dithiothreitol and 500 μM ATP. About 0.1 μg of the DNA linker mentioned above was added to the mixture, followed by addition of 6 units of T4DNA ligase. Ligation reaction was carried out at 4°C for 17 hours. *Escherichia coli* HB101 was transformed using the obtained recombinant plasmid mixture of conventional technique to obtain an Ap^R colony. A plasmid, pGKA—2 illustrated in Fig. 2 was isolated from the culture broth of the colony. The structure of pGKA—2 was confirmed by digestion with EcoRI, ClaI, HindIII, BstNI and SalI and agarose gel electrophoresis. It was confirmed by the method of Maxam-Gilbert [A.M. Maxam, et al.: Proc. Natl. Acad. Sci., 74, 560 (1977)] that the base sequence from the SD-sequence (AAGG) to the initiation codon (ATG) in the plasmid pGKA—2 is "AAGGGTA TCGATAAGCTTATG".

The human IFN—γ DNA was pGKA—2 is different from known DNAs in that the DNA has RsaI site and the ninth amino acid of the human IFN—γ polypeptide encoded by the DNA is glutamine (Gln).

Further, the synthesized DNA used above is different from the one used by P.W. Gray et al. having the following structure:

```
            m e t c y s t y r c y s
        A A T T C|A T G|T G T T A T T G T C
            G T A C A C A A T A A C A G T
```

in the underlined parts. Thus, pGKA—2 has a restriction site for BstNI, CCAGG, in the DNA region coding for human IFN—γ.

Furthermore, the length and structure between the SD sequence and ATG are important because of influence on the expression of proteins in *Escherichia coli*. The base sequence between the SD-sequence and ATG in pGKA—2 is apparently different from that in the known recombinant plasmid pIFN—γ trp48 (P.W. Gray et al.)

*Escherichia coli* containing pGKA—2 has been deposited with the FERM as *Escherichia coli* IGKA—2 (FERM P—6798) (FERM BP—496).

### Example 3

Production of interferon by *Escherichia coli* IGC—7 and IGKA—2:

*Escherichia coli* HB101 strains containing the recombinant plasmids pGC—7 and pGKA—2 obtained in Examples 1 and 2 (IGC—7 and IGKA—2, respectively) were cultured at 37°C for 18 hours in an LG medium consisting of 10 g/l tryptone, 5 g/l yeast extract, 5 g/l NaCl and 2 g/l glucose and adjusted to pH 7.0 with NaOH. Then, 0.2 ml of the culture medium was inoculated in 10 ml of an MCG medium (pH 7.2) consisting of 0.6% Na₂HPO₄, 0.3% KH₂PO₄, 0.5% NaCl, 0.1% NH₄Cl, 0.5% glucose, 0.5% casamino acid, 1 mM MgSO₄ and 4 μg/ml vitamin B₁. Culturing was carried out at 30°C for 4—8 hours. Then, 10 μg/ml indol acrylic acid (referred to as IAA hereinafter) which is an inducer of the tryptophan gene was added and culturing was

5

continued for an additional 2—12 hours. Cells were harvested by centrifugation at 8,000 rpm for 10 minutes and washed with 30 mM NaCl and 30 mM Tris-HCl (pH 7.5) buffer solution. The washed cells were suspended in 1 ml of the buffer solution mentioned above and 200 µg of lysozyme and 5 µl of 0.25 M EDTA (ethylene diamine tetraacetic acid) were added. The mixture was allowed to stand at 0°C for 30 minutes. Freezing and melting were repeated three times to disrupt the cells. The disrupted cells were centrifuged at 15,000 rpm for 30 minutes to obtain a supernatant fluid. The amount of the interferon in the supernatant was determined according to the method of Armstrong [J.A. Armstrong et al., Appl. Microbiol., Vol. 21, 723—725 (1971)] wherein Sindvis virus was used as a virus and FL cells derived from human amnion cells were used. The result is shown in Table 1.

### Table 1

| Strain | Plasmid contained | IFN-γ (unit/l) |
|---|---|---|
| IGC-7 | pGC-7 | 0 |
| IGKA-2 | pGKA-2 | $1 \times 10^{6} - 2 \times 10^{8}$ |

Reference example 1
Construction of a plasmid vector having a trp promoter:

(a) Purification of tryptophan transducing phage DNA:

A λptrp phage, λcI857trpED10 [referred to as λtrp Ed hereinafter, G.G. Miazzari et al., J. Bacteriol., Vol. 133, 1457 (1978)] was lysogenized in *Escherichia coli* JA 194 strain [F⁻, λ⁻, $r_k^-$, $m_k^-$, Δtrp E5, leu6, J. Carbon et al., Recombinant Molecules, p. 355 (1977), Raven Press]. The resultant lysogenic strain JA 194 (λtrp ED) was cultured at 42°C for 30 minutes to induce λtrp ED phages and prepare λphage lysage. The λphages were purified from the λphage lysage by the cesium chloride equilibrium density gradient centrifugation of Yamakawa et al., "Chemicals of Nucleic Acids I" Tokyo Kagaku Dojin, p. 54—61, (1974). The λphages were further purified by phenol treatment and chloroform treatment according to the method of Yamakawa et al., "Chemicals of Nucleic Acids I" Tokyo Kagaku Dojin, p. 62—65, (1974)].

(b) Cloning of the trp promoter into a plasmid:

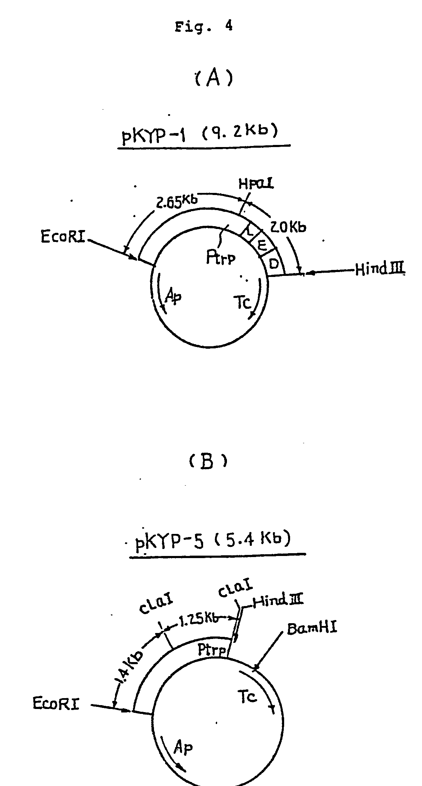

Cloning of the trp operon from λtrp Ed phage DNA was carried out in the following manner. That is, 8 µg of λtrp ED DNA was digested at 37°C for 2 hours with 16 units of EcoRI and 16 units of HindIII in 20 mM Tris-HCl (pH 7.5), 75 mM NaCl, 10 mM MgCl₂ and 5 mM dithiothreitol. Separately, 1 µg of plasmid pBR325 DNA was digested with 2 units of EcoRI and 2 units of HindIII by the same method as mentioned above (final volume: 30 µl). The reactions were stopped by heating at 65°C for 5 minutes. Then, 15 µl each of the digests were mixed and 500 µM (final concentration) ATP and 5 units of T4 DNA ligase (product of New England Biolabs) were added. The mixture was allowed to react at 4°C for 18 hours. *Escherichia coli* C600 SF strain [Cameron et al., Proc. Natl. Acad. Sci., Vol. 72, 3416 (1975)] was transformed with the plasmid mixture by the conventional method [S.N. Cohen et al., Proc. Natl., Acad. Sci. Vol. 69, 2110 (1972)] and transformants resistant to ampicillin (Apᴿ), resistant to tetracycline (Tcᴿ) and sensitive to chloramphenicol (Cmˢ) were obtained. Plasmid DNSa were isolated from the transformants of *Escherichia coli* and cleaved with EcoRI, HindIII and HpaI. One of the plasmid DNAs had the structure illustrated in Fig. 4 (A) and was named pKYP—1.

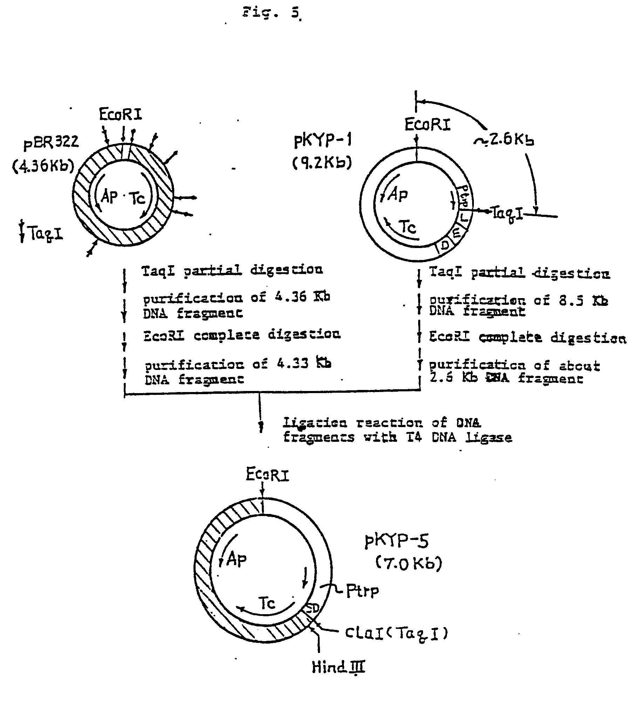

A TaqI cleavage site exists between 4 base pairs downstream from the SD sequence (AAGG) present downstream from the tryptophan promoter. By taking advantage of this fact, the plasmid pKYP—1 was digested with TaqI and EcoRI and the digest was subjected to purification by agarose gel electrophoresis to obtain a 2.6 Kb DNA fragment containing the tryptophan promoter and SD sequence. The 2.6 Kb DNA fragment was cloned in a known vector, pBR322, by the method as illustrated in Fig. 5. That is, 8 µg of pBR322 was digested at 45°C for 60 minutes with 2 units of TaqI in 100 µl of a reaction mixture comprising 10 mM Tris-HCl (pH 8.4), 6 mM MgCl₂, 100 mM NaCl and 6 mM 2-mercaptoethanol. After partial digestion with TaqI, the digest was subjected to low-gelling-temperature agarose gel electrophoresis (Lars Wieslander: Analytical Biochemistry, Vol. 98, 305 (1979)] to obtain a 4.36 Kb purified DNA fragment. About 1.5 µg of the DNA fragment was completely digested at 37°C for 3 hours with 3 units of EcoRI. About 1.0 µg of a 4.33 Kb DNA fragment was recovered by the same low gelling-temperature agarose gel electrophoresis as mentioned above. Then, 12 µg of pKYP—1 DNA was partially digested with 3 units of TaqI, the digest was subjected to low-gelling-temperature agarose gel electrophoresis to obtain about 2 µg of a 8.5 Kb purified DNA fragment and the DNA was completely digested with EcoRI by the same procedure as above to obtain about 0.5 µg of a purified 2.6 Kb DNA fragment. Then, 0.4 µg of the 4.33 Kb DNA of pBR322 and 0.25 µg of the 2.6 Kb DNA fragment of pKYP—1 thus obtained were added to 20 µl of a reaction solution consisting of 20 mM Tris-HCl (pH 7.6), 10 mM MgCl₂ and 10 mM dithiothreitol. Then, 0.5 mM ATP and 4 units of T4 DNA ligase were added to the mixture and reaction was carried out at 4°C for 18 hours. *Escherichia coli* C600 SF8 strain was transformed with the recombinant plasmid DNA obtained as above. The plasmids in the transformant having Ap$^R$ and Tc$^R$ were isolated. The plasmid DNA was digested with 6 restriction endonucleases, EcoRI, HindII, ClaI (product of Boehringer Mannheim GmbH), HpaI, HincII and BamHI to analyze the structure of the plasmid. The plasmid was recognized to have the structure illustrated in Fig. 4 (B) and was named pKYP—5.

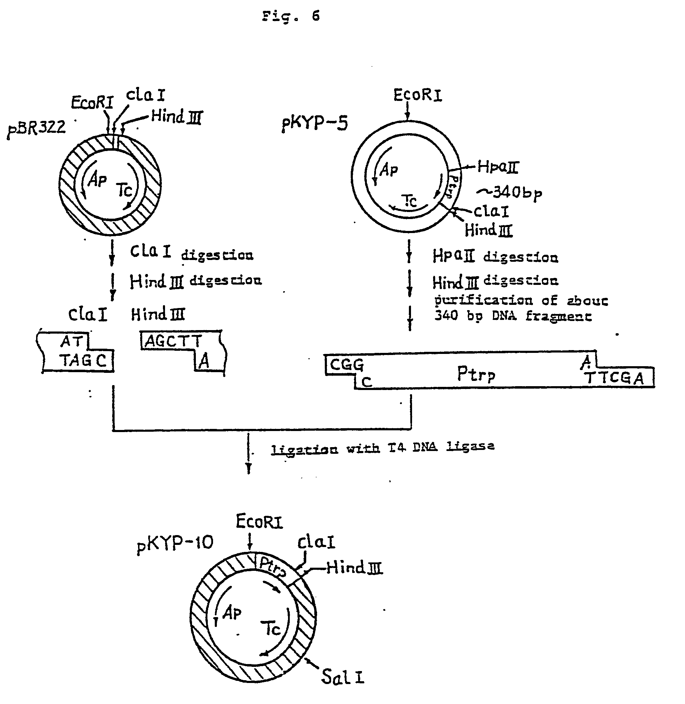

(c) Preparation of a portable promoter from the trp promoter:
The plasmid vector pKYP—5 mentioned above is applicable as a DNA introducing vector since it has a ClaI site and a HindIII site on the DNA of 1 to 20 base pairs downstream from the SD sequence. However, another ClaI site is present in pKYP—5 DNA besides the ClaI site immediately after the SD sequence and the fragment with the trp promoter obtained by cutting pKYP—5 DNA with EcoRI and HindIII is a little too large, i.e. 2.65 Kb. For convenience of use, pKYP—5 was improved by the process as illustrated in Fig. 6 to obtain a plasmid having a DNA fragment containing a shorter tryptophan promoter. That is, pKYP—5 DNA was digested with HpaII and HindIII and the digest was purified to obtain a DNA fragment of about 340 bp. The fragment was inserted into the pBR322 digested with ClaI and HindIII as illustrated in Fig. 6 to obtain pKYP—10. The structure of pKYP—10 was determined by digestion with EcoRI, ClaI, HindIII and HpaI and agarose gel electrophoresis.

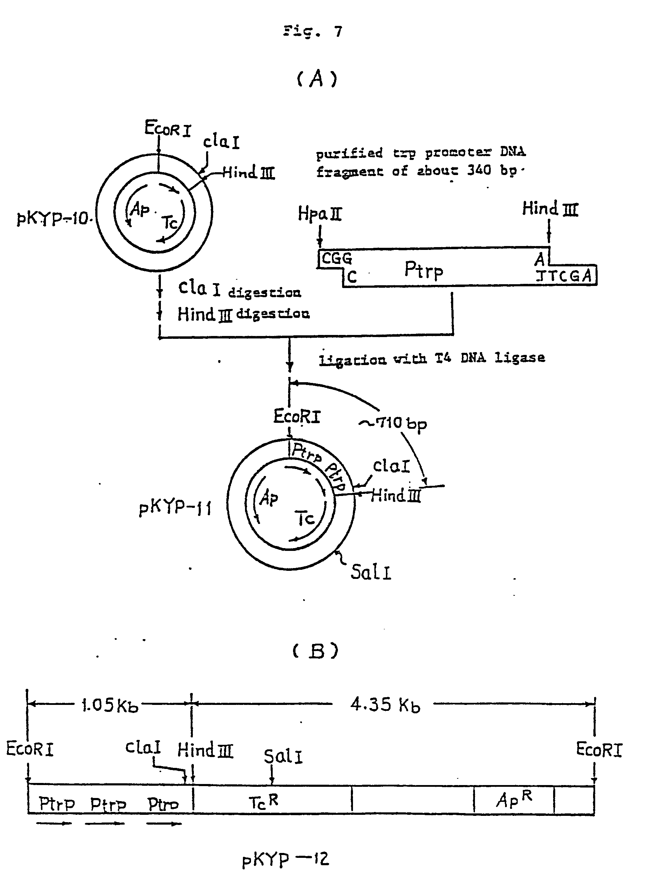

(d) Connection of two or more trp promoters:
In order to construct a plasmid vector having a stronger promoter activity, two trp promoters were inserted into the vector. That is, the DNA fragment of about 340 bp containing the trp promoter mentioned in step (c) above was inserted into the pKYP—10 digested with ClaI and HindIII to obain pKYP—11 as illustrated in Fig. 7 (A). The same procedure was repeated to construct pKYP—12 illustrated in Fig. 7 (B) wherein three trp promoters were connected at the some orientation. The structure of pKYP—12 was confirmed by digestion with EcoRI, ClaI, HindIII and HpaI.

**Claims**

1. A recombinant plasmid containing a tryptophan promotor and downstream thereof a DNA fragment encoding a human interferon-γ polypeptide having the amino acid sequence illustrated in Fig. 3.
2. A recombinant plasmid according to claim 1, which is named pGC—7 (FERM BP—497) or pGKA—2 (FERM BP—496).
3. A process for producing a human interferon-γ polypeptide having the amino acid sequence illustrated in Fig. 3 which comprises culturing in a medium of microorganism tranformed with a recombinant plasmid according to claim 1 or 2, accumulating said interferon-γ polypeptide in the cultured cells and recovering the interferon-γ polypeptide therefrom.
4. The process according to claim 3, wherein the microorganism belongs to *Escherichia coli*.
5. A microorganism containing a recombinant plasmid according to claim 1 or 2.
6. The microorganism according to claim 5, which belongs to *Escherichia coli*.
7. *Escherichia coli* IGC—7 (FERM P—6814) (FERM BP—497).
8. *Escherichia coli* IGKA—2 (FERM P—6798) (FERM BP—496).

**Patentansprüche**

1. Rekombinantes Plasmid enthaltend einen Tryptophan-Promoter und stromabwärts davon ein DNAFragment, das für ein menschliches Interferon-γ-Polypeptid mit der in Fig. 3 gezeigten Aminosäuresequenz codiert.
2. Rekombinantes Plasmid nach Anspruch 1, das als pGC—7 (FERM BP—497) oder pGKA—2 (FERM BP—496) bezeichnet wird.
3. Verfahren zur Herstellung eines menschlichen Interferon-γ-Polypeptids mit der in Fig. 3 gezeigten Aminosäuresequenz, bei dem man in einem Medium einen mit einem rekombinanten Plasmid nach Anspruch 1 oder 2 transformierten Mikroorganismus kultiviert, das Interferon-γ-Polypeptid in den kultivierten Zellen akkumuliert und das Interferon-γ-Polypeptid daraus isoliert.
4. Verfahren nach Anspruch 3, bei dem der Mikroorganismus zu Escherichia coli gehört.
5. Mikroorganismus enthaltend ein rekombinantes Plasmid nach Anspruch 1 oder 2.
6. Mikroorganismus nach Anspruch 5, der zu Escherichia coli gehört.
7. Escherichia coli IGC—7 (FERM P—6814) (FERM BP—497).
8. Escherichia coli IGKA—2 (FERM P—6798) (FERM BP—496).

**Revendications**

1. Plasmide recombinant qui contient un promoteur tryptophane et en aval de celui-ci, un fragment d'ADN codant pour un polypeptide du γ-interféron humain comportant la séquence en acides aminés illustrée sur la figure 3.

2. Plasmide recombinant selon la revendication 1, qui est appelé pGC—7 (FERM BP—497) ou pGKA—2 (FERM BP—496).

3. Procédé pour la production de polypeptide du γ-interféron humain comportant la séquence en acides aminés illustrée sur la figure 3, qui consiste à cultiver dans un milieu un micro-organisme transformé avec un plasmide recombinant selon la revendication 1 ou 2, à accumuler le polypeptide du γ-interféron dans les cellules cultivées et à récupérer le polypeptide du γ-interféron à partir de celles-ci.

4. Procédé selon la revendication 3, dans lequel le micro-organisme appartient à *Escherichia coli.*

5. Micro-organisme qui contient un plasmide recombinant selon la revendication 1 ou 2.

6. Micro-organisme selon la revendication 5, qui appartient à *Escherichia coli.*

7. *Escherichia coli* IGC—7 (FERM P—6814) (FERM BP—497).

8. *Escherichia coli* IGKA—2 (FERM P—6798) (FERM BP—496).

Fig. 1

EP 0 121 157 B1

F i g . 2

pKYP-10(4.7Kb)

pGC-7(6.0 Kb)

Synthetic DNA

Hind III
Sal I

18-mer    15-mer

BstN I
Sal I

Purification
of a fragment
of about 4.1 Kb

5'-Phospho-
rylation

Purification
of a fragment
of about 1125 bp

Annealing

18bp    BstNI    1,125bp    Sal I

Hind III    15bp

Ligation of the DNA fragments
with T4 DNA ligase

pGKA-2 (5.25Kb)

2

## Fig. 3

CACATTGTTCTGATCATCTGAAGATCAGCTATTAGAAGAGAAAGATCAGTTAAGTCCTTTGGACCTGATCAGCTTGATACAAGAACTACTGATTT

```
                                    met lys tyr thr ser tyr ile leu ala phe gln leu cys ile val leu
CAACTTCTTTGGCTTAATTCTCTCGGAAACG ATG AAA TAT ACA AGT TAT ATC TTG GCT TTT CAG CTC TGC ATC GTT TTG
```

```
gly ser leu gly CYS TYR CYS GLN ASP PRO TYR VAL GLN GLU ALA GLU ASN LEU LYS LYS TYR PHE ASN ALA
GGT TCT CTT GGC TGT TAC TGC CAG GAC CCA TAT GTA CAA GAA GCA GAA AAC CTT AAG AAA TAT TTT AAT GCA
                                            RsaI
```

```
GLY HIS SER ASP VAL ALA ASP ASN GLY THR LEU PHE LEU GLY ILE LEU LYS ASN TRP LYS GLU GLU SER ASP
GGT CAT TCA GAT GTA GCG GAT AAT GGA ACT CTT TTC TTA GGC ATT TTG AAG AAT TGG AAA GAG GAG AGT GAC
```

```
ARG LYS ILE MET GLN SER GLN ILE VAL SER PHE TYR PHE LYS LEU PHE LYS ASN PHE LYS ASP ASP GLN SER
AGA AAA ATA ATG CAG AGC CAA ATT GTC TCC TTT TAC TTC AAA CTT TTT AAA AAC TTT AAA GAT GAC CAG AGC
```

```
ILE GLN LYS SER VAL GLU THR ILE LYS GLU ASP MET ASN VAL LYS PHE PHE ASN SER ASN LYS LYS LYS ARG
ATC CAA AAG AGT GTG GAG ACC ATC AAG GAA GAC ATG AAT GTC AAG TTT TTC AAT AGC AAC AAA AAG AAA CGA
```

```
ASP ASP PHE GLU LYS LEU THR ASN TYR SER VAL THR ASP LEU ASN VAL GLN ARG LYS ALA ILE HIS GLU LEU
GAT GAC TTC GAA AAG CTG ACT AAT TAT TCG GTA ACT GAC TTG AAT GTC CAA CGC AAA GCA ATA CAT GAA CTC
```

```
ILE GLN VAL MET ALA GLU LEU SER PRO ALA ALA LYS THR GLY LYS ARG LYS ARG SER GLN MET LEU PHE ARG
ATC CAA GTG ATG GCT GAA CTG TCG CCA GCA GCT AAA ACA GGG AAG CGA AAA AGG AGT CAG ATG CTG TTT CGA
```

```
GLY ARG ARG ALA SER GLN
GGT CGA AGA GCA TCC CAG TAA TGGTTGTCCTGCCTGCAATATTTGAATTTTAAATCTAAATCTATTTATTAATATTTAACATTATTTA
```

TATGGGGAATATATTTTTAGACTCATCAATCAAATAAGTATTTATAATAGCAACTTTTGTGTAATGAAAATGAATATCTATTAATATATGTATTA

TTTATAATTCCTATATCCTGTGACTGTCTCACTTAATCCTTTGTTTTCTGACTAATTAGGCAAGGCTATGTGATTACAAGGCTTTATCTCAGGGG

CCAACTAGGCAGCCAACCTAAGCAAGATCCCATGGGTTGTGTGTTTATTTCACTTGATGATACAATGAACACTTATAAGTGAAGTGATACTATCC

AGTTACTA CCCCCCC''''

Fig. 4

(A)

pKYP-1 (9.2 Kb)

(B)

pKYP-5 (5.4 Kb)

Fig. 5

Fig. 6

## Fig. 7

### ( A )

purified trp promoter DNA fragment of about 340 bp·

### ( B )

pKYP—12